# EUROPEAN PATENT SPECIFICATION

(11) **EP 3 777 970 B1**
(45) Date of publication and mention of the grant of the patent: **11.10.2023**
(21) Application number: 19782107.7
(22) Date of filing: 25.03.2019
(51) Int. Cl.: A61N 1/39, A61B 5/361

(54) **DEFIBRILLATOR FOR REDUCING TIME TAKEN FROM POWERING-ON UP TO ELECTRICAL SHOCK INDICATION**
DEFIBRILLATOR ZUR REDUZIERUNG DER ZEIT VOM EINSCHALTEN BIS ZUR ELEKTROSCHOCKANZEIGE
DÉFIBRILLATEUR POUR RÉDUIRE LE TEMPS PRIS À PARTIR DE LA MISE SOUS TENSION JUSQU'À UNE INDICATION DE CHOC ÉLECTRIQUE

(30) Priority: 02.04.2018 KR 20180037911; 31.12.2018 KR 20180174188; 10.01.2019 KR 20190003533
(43) Date of publication of application: 17.02.2021
(73) Proprietor: Mediana Co., Ltd., Wonju-si, Gangwon-do 26365 (KR)
(72) Inventor: LEE, Sung Ho, Wonju-si Gangwon-do 26409 (KR); LEE, Hee Tack, Wonju-si Gangwon-do 26388 (KR); HWANG, Jeong Gi, Wonju-si Gangwon-do 26344 (KR); KANG, Dong Won, Wonju-si Gangwon-do 26482 (KR)
(74) Representative: Gulde & Partner
(86) International application number: PCT/KR2019/003455
(87) International publication number: WO 2019/194444

(56) References cited:
- WO-A1-2008/036869
- JP-A- 2008 543 479
- JP-A- 2017 538 493
- JP-B2- 2 815 266
- KR-A- 20140 126 649
- KR-A- 20170 122 680
- US-A1- 2003 095 648
- US-A1- 2009 005 827

## Description

### [Technical Field]

The present invention relates to a defibrillator that enables electrocardiogram (ECG) analysis and high-voltage charging to proceed simultaneously from the time point at which the defibrillator power is turned on to reduce the time it takes to deliver the initial shock and enable early defibrillation, thereby raising the probability of resuscitating the patient.

### [Prior Art]

In the event of a heart attack, blood stops circulating throughout the body, and serious brain damage or even death may occur unless immediate action is taken to restart the heart. The brain, in particular, starts to undergo irreparable damage even if blood supply is stopped even for 4 to 5 minutes, and past 6 minutes, the functions of the brain and all other organs may stop and lead to a loss of life.

Cardiopulmonary resuscitation (CPR) is an emergency treatment method of artificially circulating blood and starting respiration in the event of a heart attack (cardiac arrest) and must be performed as an emergency procedure if a sudden cardiac arrest in which the heart suddenly stops occurs. CPR refers to the act of performing artificial ventilation and cardiac compression (heart massage) in combination. Generally, without endotracheal intubation, it is recommended that CPR be performed, in the case of adults, at a 30:2 ratio for chest compressions to artificial ventilation, respectively (a 15:2 ratio in the case of children), and thus, in the case of adults, two artificial ventilations should be performed after thirty consecutive chest compressions. CPR generally refers to performing thirty consecutive chest compressions and two artificial ventilations thereafter. Hereinafter, in relation to the present invention, thirty consecutive chest compressions and two artificial ventilations for adults, or consecutive chest compressions for two minutes (thirty consecutive chest compressions) are expressed as one set of CPR, and in the case of children, fifteen consecutive chest compressions and two artificial ventilations, or consecutive chest compressions for two minutes are expressed as one set of CPR.

Automated external defibrillator (AED) is equipment necessary for the process of performing CPR and a device for removing ventricular fibrillation. Generally, an AED is designed to allow the user to check the heart condition of the patient and automatically give an electric shock to the patient.

In the event of a heart attack, blood circulation in the body stops, and serious brain damage or death may occur without immediate treatment. The brain, in particular, may be permanently damaged, if the blood supply is stopped for just 4 to 5 minutes. While normal CPR restores blood flow to 20% of the normal rate, 100% recovery is possible using an AED, and thus, an AED can increase the survival rate of cardiac arrest patients by up to 70%.

The human heart has a command system that acts on the myocardium in a certain order in order to effectively release blood. That is, the heart pumps blood through the arteries by contracting and expanding repeatedly in a certain order. If the heart does not move in a predetermined order, it cannot efficiently supply blood to the arteries. Therefore, the heart supplies blood to the arteries while repeatedly undergoing contraction and expansion in a certain order, and CPR using an AED, which causes the heart to move in a predetermined order, significantly affects the survival rate after an accident.

In the case of one embodiment of the existing art, analysis during compressions with a fast reconfirmation (ADC-FR) technology is applied for electrocardiogram (ECG) analysis during CPR, and high-voltage charging is performed after CPR to shorten the time it takes for the ECG analysis. In other words, it reduces the delay until the next electric shock after CPR than before performing CPR. However, although it applies a method for reducing the time from analysis to electric shock by analyzing the ECG during CPR, it is unable to shorten the time between turning on the power and the delivery of the first electric shock. Thus, it may not fall under the category of early defibrillation, but it may minimize disruptions in CPR.

As for another embodiment of the existing technology, high-voltage charging is performed during CPR, and ECG analysis is performed after CPR in order to reduce the time required for high voltage charging. As the case with the embodiment described above, this also cannot shorten the time it takes to deliver the first electric shock. Even in this case, it does not fall under the category of early defibrillation, yet it can minimize disruptions in CPR.

Generally, when using a defibrillator, after the power is turned on, the defibrillator equipment performs a self-check, an ECG rhythm analysis to check whether it is shockable, high voltage charging, etc., and defibrillation is performed by pressing the electric shock delivery button. The shorter the time it takes from when the defibrillator device is turned on to the electric shock delivery button is pressed, the higher the probability of resuscitation 1 will be. Normally, early defibrillation is reported to increase the resuscitation rate, and if defibrillation is not performed after a heart attack, the resuscitation rate is decreased by 10% per minute, which means that death results after 10 minutes. That is, when a heart attack occurs, it is necessary to take immediate action, and early defibrillation has a significant impact on the resuscitation rate.

In the case of prior art, there were methods that reduce the time it would take to delivery an electric shock by conducting an ECG analysis or performing high-voltage charging during CPR as a way to reduce the time from ECG analysis to an electric shock delivery. However, the time is shortened when the ECG analysis is performed again following the process of analysis, charging, electric shock delivery, and CPR, and it does not lead to early defibrillation.

The present invention relates to a defibrillator configured to shorten the time it takes to deliver an electric shock to a patient by conducting ECG analysis and high-voltage charging at the same time, and it shortened the time for delivering an electric shock by conducting high-voltage charging at the same time as ECG analysis immediately after the power is turned on, rather than shortening the time after the initial CPR, as is the case with prior art.

The present invention relates to a defibrillator configured for simultaneous performance of ECG analysis and high-voltage charging from the point of time at which the defibrillator is turned on, thereby reducing the time it takes to deliver the first electric shock and allowing early defibrillation, which in turns increases the probability of resuscitating the patient.

That is, according to the present invention, ECG analysis and high-voltage charging are performed simultaneously so as to reduce the time it takes to deliver an electronic shock to the patient. The time is shortened not after the CPR is already performed once, as in the prior art, but the time it takes to deliver an electric shock delivery is shortened by allowing ECG analysis and high-voltage charging to proceed at the same time as soon as the power is turned on.

As a prior art, Japanese Patent Application Publication No. 2008-508976 relates to a method of determining the timing of providing a defibrillation shock from an automatic external defibrillator (AED). The ECG signal prior to the CPR is analyzed to prepared said AED to provide a defibrillation shock.

As another prior art, U.S. Patent No. US07904152 relates to an art in which a medical device is activated and the first analysis of the first set of data signals from the patient detected by the medical device is carried out, provided that if the first analysis shows that the first set of data signals satisfies the first set of criteria, the energy storage device starts being charged upon completion of the first analysis. Also, regardless of the result of the first analysis, a second analysis is performed on the second set of data signals from the patient, provided that in the second analysis, if the second set of data signals is determined to satisfy a second set of criteria that differs from the first set of criteria, said energy storage device is charged to a level enabling the medical device to deliver electrotherapy, and electrotherapy is provided regardless of the result of the first analysis.

Japanese Patent Application Publication No. 2008-508976 and US Patent US07904152 relate to art in which charging is determined based on the biosignal such as an ECG signal. Thus, early defibrillation is difficult with the application of the art under Japanese Patent Application Publication No. 2008-508976 and US Patent US07904152.

As another prior art, Korean Patent Application Publication No. 10-2017-0122680 relates to an art wherein, when a start signal is input from the start/stop key, the charging unit is operated to charge the electric shock power unit to a baseline, but detection of the ECG signal does not occur at the same time. Early defibrillation cannot occur with the application of the art under Korean Patent Application Publication No. 10-2017-0122680. A defibillator according to the preamble of claim 1 is known from US2009005827 A1.

Generally, the ECG signal is detected to determine whether to give an electric shock. Since the noise generated during high-voltage charging affects the ECG analysis, ECG detection and voltage charging are performed separately, which takes time, and this is what renders early defibrillation difficult.

### [Detailed Description of the Invention]

The invention is defined in claim 1. Any methods disclosed hereinafter do not form part of the scope of the invention and are mentioned for illustrative purposes only.

### [Technical Issue to Be Addressed]

The technical objective the present invention is to provide a defibrillator that enables ECG analysis and high-voltage charging to proceed simultaneously from the time the defibrillator is turned on so as to reduce the time it takes to deliver the initial electric shock and enable early defibrillation, thereby raising the probability of resuscitating the patient.

### [Technical Means to Address the Issue]

In order to address the above issue, the present invention relates to a defibrillator comprised of an ECG detection unit that detects and amplifies ECG signals, removes noise, and converts it into a digital signal; an operation processing unit that analyzes the ECG signal received from the ECG detection unit to check for ventricular fibrillation and to generate an electric shock signal if ventricular fibrillation is present; an electric shock unit that applies high energy to a defibrillation electrode upon receiving an electric shock signal from the operation processing unit so as to exert an electric shock, wherein the operation processing unit, as soon as the power switch of the key input unit is turned on, analyzes ECG signals received from the ECG detection unit and at the same time, transmits a signal (namely, charging instruction signal) to charge to the energy charging unit of the electric shock unit, thereby having the energy charging unit to charge the energy of the electric shock power unit to a preset baseline.

The operation processing unit first analyzes the ECG signal received from the ECG detection unit to first determine whether ventricular fibrillation is present, and if it is determined that there is ventricular fibrillation, it judges that it is time to deliver an electric shock and provides an electric shock delivery guidance through the speaker. From the ECG signal during the time from the point at which an electric shock is judged necessary to the time point at which an electric shock delivery guidance is provided through the speaker, it is determined whether there is ventricular fibrillation for the second time, and if it is judged that there is ventricular fibrillation, an electric shock signal is generated and sent to the electric shock unit for the electric shock unit to deliver an electric shock.

The operation processing unit, when it is judged that it is not ventricular fibrillation during the second assessment for presence of ventricular fibrillation, cancels the prior judgment that an electric shock is necessary and determines whether such cancellation has occurred three times in succession. If such cancellation has occurred three times in succession, the operation processing unit generates a signal to discharge and transmits it to the energy discharging unit for the energy discharging unit to discharge the power charged in the energy charging unit.

After an electric shock is performed by the electric shock unit, or after the operation processing unit transmits a signal to discharge to the energy discharge unit, the preset CPR time duration (CPR time interval) begins, and the operation processing unit, after the CPR time CPR time duration ends, receives ECG signal from the ECG detection unit for analysis and, at the same time, transmits the signal to charge to the energy charging unit of the electric shock unit for the energy charging unit to charge the energy of the electric shock power unit to a preset baseline.

The CPR time duration may be 2 minutes.

The operation processing unit first analyzes the ECG signal received from the ECG detection unit to first determine whether there is ventricular fibrillation and, if it is determined that it is not ventricular fibrillation, generates a signal to discharge to be transmitted to the energy discharge unit for the energy discharge unit to discharge the power charged in the energy charging section from the inside the equipment via internal discharge.

The operation processing unit, when it is judged that it is not ventricular fibrillation during the second assessment for presence of ventricular fibrillation, cancels the prior judgment that an electric shock is necessary and determines whether such cancellation has occurred three times in succession. If such cancellation has not occurred three times in succession, the operation processing unit receives ECG signal from the ECG detection unit for analysis and, at the same time, transmits the signal to charge to the energy charging unit of the electric shock unit for the energy charging unit to charge the energy of the electric shock power unit to a preset baseline.

The ECG detection unit, for the purpose of simultaneously analyzing ECG signal and charging energy, further comprises an ECG signal filter unit that removes noise caused by high voltage charging from the ECG signal.
The baseline may vary depending on whether the patient is a child or adult or the age or weight of the patient. That is, the baseline differs between children or adults, and it may be selected by the user in consideration of the patient's weight, age, etc.

In addition, the defibrillator of the present invention is characterized by a charging step, wherein the energy the energy charging unit charges the energy of the electric shock power unit until the preset baseline according to a signal (namely, charging instruction signal) to charge from the operation processing unit, when power is input from the power switch of the key input unit; an ECG analysis step, which is carried out at the same time as the charging step when power is input from the power switch of the key input unit, wherein the operation processing unit analyzes the ECG signal received from the ECG detection unit to first determine whether ventricular fibrillation is present; an electric shock timing determination step, wherein the operation processing unit determines whether to apply electric shock depending on the presence of ventricular fibrillation after the ECG analysis step, thereby setting the electronic shock flag and providing an electric shock delivery guidance via the sound output unit.

The defibrillator further comprises an electric shock cancellation determination step, wherein, in case it is judged that electric shock is necessary in the electric shock timing determination step, it is judged for the second time by the operation processing unit whether there is ventricular fibrillation, based on the ECG signal during from the time point at which an electric shock flag is set in the electric shock timing determination step to the time point at which an electric shock delivery guidance is provided through the speaker, and the electric shock flag is reset to cancel the electric shock if it is judged that there is no ventricular fibrillation.

The defibrillator further includes a three successive cancellation determination step, wherein, in case the delivery of an electric shock is canceled as it is judged that there is no ventricular fibrillation during the second assessment for presence of ventricular fibrillation by the operation processing unit in the shock cancellation determination step, it is judged whether the cancellation of an electric shock delivery has occurred three times in succession; and a discharging step, wherein, in case it is judged that it was not the right timing for an electric shock in the electric shock timing determination step or in case it is judged that electric shock cancellation occurred three times in succession in the three successive cancellation determination step, a signal (namely, discharge instruction signal) to discharge is generated and transmitted to the energy discharge unit.

The defibrillator further comprises an electric shock step, wherein, in case it is judged that there is ventricular fibrillation during the second assessment for presence of ventricular fibrillation in the shock cancellation determination step, the operation processing unit generates an electric shock signal and transmits it to the electric shock unit for the electric shock unit to exert an electric shock.

In the three successive cancellation determination step, if it is determined that cancellation of the electric shock did not occur three times in succession, the operation processing unit performs the charging and ECG analysis steps simultaneously.

The defibrillator further includes a CPR step, wherein the CPR time duration (CPR time interval) begins after the electric shock step or discharging step and the operation processing unit waits for the end of the CPR time.
After the CPR step, the operation processing unit performs the charging step and the ECG analysis step simultaneously.

In the ECG analysis step, the operation processing unit removes noise caused by high voltage charging occurring during the charging step from the ECG signal received from the ECG detection unit and analyzes the ECG signal from which the noise is removed to first determine whether there is ventricular fibrillation.

### [Effects of the Invention]

According to the defibrillator of the present invention, ECG analysis and high voltage charging are simultaneously performed from the time point at which the defibrillator is turned on, thereby reducing the time it takes to deliver the initial electric shock and enabling early defibrillation to raise the probability of resuscitating the patient.

Also, the present invention is configured to simultaneously perform ECG analysis and charging (10 seconds) after CPR to reduce the time it takes until the next electric shock. The ECG signal filter unit (not shown) of the present invention, in particular, is configured to filter noise generated during high voltage charging so that, even if charging and ECG analysis are performed simultaneously, noise generated during high voltage charging does not affect the ECG analysis.

Previously, the time required to turn on the defibrillator and charge it after the initial ECG analysis has been about 20 seconds, but the present invention enables simultaneous performance of ECG analysis and charging to shorten the duration to about 10 seconds, thereby raising the probability of resuscitating emergency patients.

In addition, in the present invention, the voltage charged in the high voltage capacitor is not discharged immediately, even when the ECG waveform returns to normal during analysis or when an electrical shock is canceled due to a sudden change in bioimpedance, and is instead maintained so that energy is discharged via internal discharge in the event that the electric shock is canceled three consecutive times, thereby reducing the time required recharging.

### [Brief Descriptions of the Figures]

FIG. 1 is a block diagram schematically showing the configuration of a defibrillator having an operating method which is not part of the present invention that shortens the time from turning on the power to giving a direction for an electric shock.
FIG. 2 is a flowchart schematically showing an operating method which is not part of the present invention that shortens the time from turning on the power to giving a direction for an electric shock.

### [Specific Details for Implementation of the Invention]

Hereinafter, a defibrillator of the present invention that shortens the time from turning on the power to giving instruction signal for an electric shock will be described in detail with reference to the attached figures.

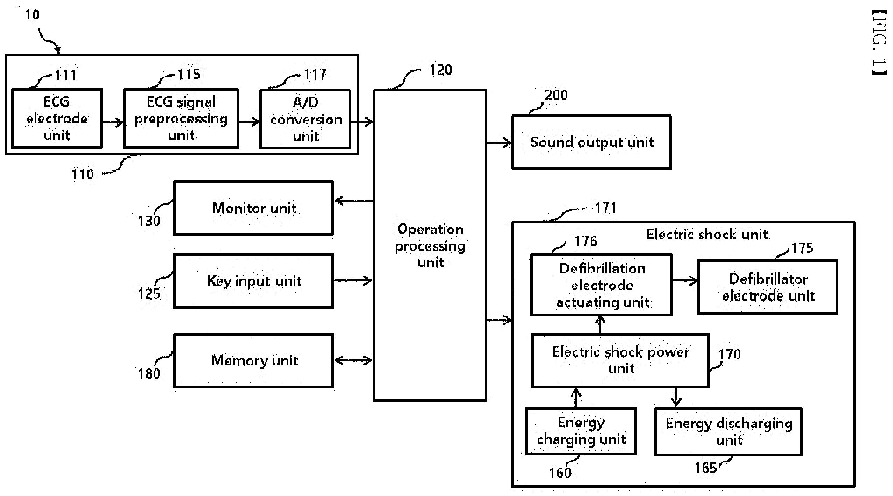

FIG. 1 is a block diagram schematically showing the configuration of a defibrillator having an operating method which is not part of the present invention that shortens the time from turning on the power to giving a direction for an electric shock. It includes an ECG detection unit (110), operation processing unit (120), key input unit (125), energy charging unit (160), energy discharging unit (165), electric shock power unit (170), defibrillation electrode actuating unit (176), defibrillator electrode unit (175), and sound output unit (200).

The ECG detection unit (110) is a means for detecting a patient's ECG to assess his/her condition, and includes an ECG electrode unit (111), ECG signal preprocessing unit (115), and A/D conversion unit (117).

The ECG signal preprocessing unit (115) includes an ECG signal amplification unit (not shown) and ECG signal filter unit (not shown), amplifies the ECG signal detected by the ECG electrode (not shown), and removes noise. The ECG signal filter unit (not shown), in particular, is configured to remove noise generated during high voltage charging. That is, in the present invention, noise generated during high voltage charging does not affect the ECG analysis.

The A/D conversion unit (117) converts the ECG signal received from the ECG signal preprocessing unit (115) into a digital signal and transmits it to the operation processing unit (120).

The operation processing unit (120) receives the ECG signal from the ECG signal preprocessing unit (115) when the power switch (not shown) of the key input unit (125) is turned on, and at the same time, sends a signal (namely, charging instruction signal) to charge to the energy charging unit (160). That is, in the present invention, the power of the defibrillator (10) is turned on, and at the same time, ECG analysis and charging are performed simultaneously.

In addition, the operation processing unit (120) performs ECG analysis and charging simultaneously after the CPR time duration (CPR time interval) (i.e., the time duration during which CPR is performed), thereby reducing the time required until the next electric shock.

Here, the CPR time duration is the time required for one set of CPR. One set of CPR consists of thirty consecutive chest compressions and two artificial ventilations in the case of adults, fifteen consecutive chest compressions and two artificial ventilations in the case of children, or consecutive chest compressions for two minutes. One set of CPR lasts about two minutes. The operation processing unit (120) may output an alarm or sound to indicate the need to perform one set of CPR during the CPR time. CPR in the present invention does not fall within the scope that is limited to the present invention, and thus a detailed description thereof will not be provided here.

In other words, when the power switch (not shown) of the key input unit (125) is turned on, the operation processing unit (120) receives the ECG signal from the ECG signal preprocessing unit (115) and performs ECG analysis at the same time, while sending a signal to charge to the energy charging unit (160) to charge energy. During ECG analysis, the operation processing unit (120) analyzes the ECG signal that has been received in order to obtain certain parameters such as the RR interval and QRS power spectrum, and these parameters are applied to determine whether the ECG is indicating ventricular fibrillation, arrhythmia, or normal heart activity. The operation processing unit (120) determines whether there is ventricular fibrillation (in other words, shockable rhythm) to judge whether an electric shock is necessary (namely, whether it is electric shock time point).

If the operation processing unit (120) determines that there is no ventricular fibrillation, and an electric shock is judged unnecessary (in other words, the operation processing unit (120) judge that it is not the electric shock time point), an signal (namely, discharge instruction signal) to internal discharge will be transmitted to the energy discharging unit (165), and the voltage charged in the high voltage capacitor of the energy discharging unit (165) will be discharged via internal discharge. Then, after waiting for the CPR time duration (i.e. the time taken for one set of CPR) to end, the operation processing unit (120) returns to the initial state after the end of the CPR time, receives the ECG signal from the ECG signal preprocessing unit (115), performs ECG analysis, and at the same time, sends a signal (charging instruction signal) to charge to the energy charging unit (160) to charge energy.

If the operation processing unit (120) determines that there is ventricular fibrillation, and an electric shock is judged necessary (in other words, if the operation processing unit (120) judged that it is the electric shock time point), an electric shock delivery guidance is provided through the sound output unit (200). And then the operation processing unit (120) obtains the ECG parameters from after the time it was determined that it was the electric shock time point, thereby judging whether the ECG determined to indicate ventricular fibrillation at the time of determining the electric shock time point is deemed to be a normal ECG. At this time, if it is still judged to indicate ventricular fibrillation, an electric shock control signal will be transmitted to the electric shock unit (171), and the electric shock unit (171) will apply an electric shock via the defibrillator electrode unit (175) with energy that has already been charged. When the electric shock is over, the operation processing unit waits for the CPR time duration (i.e. the time it takes for one set of CPR) to end. In addition, the operation processing unit (120) returns to the initial state after the end of the CPR time duration, receives the ECG signal from the ECG signal preprocessing unit (115), performs ECG analysis, and at the same time, sends a signal (charging instruction signal) to charge to the energy charging unit (160) to charge energy.

If the operation processing unit (120) determines that the electrocardiogram parameters, after determining the electric shock timing (namely, after it is judged to be the electric shock time point), indicate normal heart activity (i.e. the ECG waveform returns to normal in the ECG analysis after determining the electric shock timing) or if an electric shock is not possible due to a sudden change in the bioimpedance, the electric shock will be canceled. Then, by counting the cancellation with the successive cancellation counter (not shown), if there have been three consecutive electric shock cancellations, an signal (discharge instruction signal) to internal discharge will be transmitted to the energy discharging unit (165) for the voltage charged in the high voltage capacitor of the energy discharging unit (165) to be discharged via internal discharge. Then, after waiting for the CPR time duration (i.e. the time taken for one set of CPR) to end, the operation processing unit (120) returns to the initial state after the end of the CPR time duration, receives the ECG signal from the ECG signal preprocessing unit (115), performs ECG analysis, and at the same time, sends a signal (charging instruction signal) to charge to the energy charging unit (160) to charge energy.

That is, after transmitting an signal to internal discharge to the energy discharging unit (165), or after an electric shock is exerted by transmitting an electric shock control signal to the electric shock unit (171), the CPR time duration begins, and the operation processing unit (120) waits for the end of the CPR time duration. When the CPR time duration ends, the operation processing unit (120) simultaneously proceeds with ECG analysis and charging again, and the subsequent steps proceed as described above.

The operation processing unit (120), during the CPR time, may transmit a guide sound signal related to CPR to the sound output unit (200). The aforementioned guide sound signal may be an alarm signal indicating the time point to start chest compressions and a sound signal indicating the time point to start artificial ventilation.

The operation processing unit (120) may be comprised of a microprocessor, computer, microcontroller, etc.

The key input unit (125) includes a power switch (not shown), a manual electric shock switch (not shown), etc. In some cases, it may further include a child or adult selection button.

The electric shock unit (171) is a means for applying an electric shock and is comprised of an electric shock power unit (170), defibrillation electrode actuating unit (176), defibrillator electrode unit (175), energy charging unit (160), and energy discharging unit (165).

The energy charging unit (160) is a means for charging the electric shock power unit (170), and when a signal to charge is received from the operation processing unit (120), it charges to a high voltage used for an electric shock. Depending on the child or adult or the age or weight, the charging level or baseline (reference value) may be different.

Preferably, the charging baseline (charging level) should be provided with a charging baseline for adults and charging baseline for children, and it can be configured that the user can take the patient's age or weight into consideration at the beginning of use so that either the charging baseline for adults and charging baseline for children.

The energy discharging unit (165) is a means to discharge electric voltage from the electric shock power unit (170). When a signal (discharge instruction signal) to discharge is received from the operation processing unit (120), the voltage in the high voltage capacitor of the energy charging unit (160) is discharged via internal discharge.

The electric shock power unit (170) is a power unit that stores high-voltage energy that gets applied to the defibrillator electrode unit (175) for an electric shock and is charged by the energy charging unit (160) and discharged by the energy discharging unit (165). It may be comprised of a high-voltage capacitor or a rechargeable battery.

The defibrillation electrode actuating unit (176) applies high energy (high-voltage current, high voltage) from electric shock power unit (170) to the defibrillator electrode unit, according to the electric shock signal of the operation processing unit (120) or when the user turns on the manual electric shock switch.

The defibrillator electrode unit (175) is equipped with a left electrode pad and a right electrode pad and applies a high-energy electric shock to the patient's heart.

The monitor unit (130) displays and monitors ECG analysis results, such as ECG signals, RR intervals, QRS power spectrum, and ventricular fibrillation, received from the operation processing unit (120). Also, it displays emergency situations, such as when the degree of CPR is beyond the prescribed standard scope.

The memory unit (180) stores ECG analysis results, such as ECG signals, RR intervals, QRS power spectrum, and ventricular fibrillation, for example, received from the operation processing unit (120). Also, it stores the charging baseline for adults and the charging baseline for children.

The sound output unit (200) may consist of an alarm, a speaker, etc.

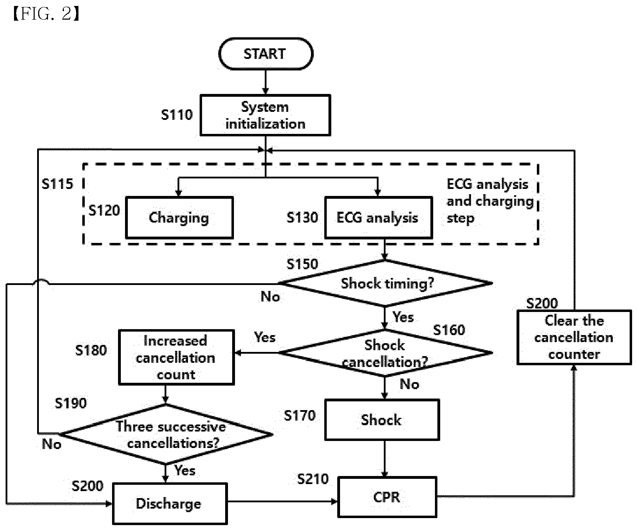

FIG. 2 is a flowchart schematically showing an operating method of the present invention that shortens the time from turning on the power to giving a direction for an electric shock.

In the initialization step, when power is inputted from the power switch of the key input unit (125), the operation processing unit (120) initializes the counter, flag, etc. (S110).

In the charging step, after the initialization step (S110), the energy charging unit (160) checks (judges) the current residual energy (power source for charging) of the electric shock power unit (170) according to the signal (charging instruction signal) to charge of the operation processing unit (120) and charges the device until the residual energy is equal to the preset baseline (S120). The baseline may vary depending on whether the patient is a child or an adult or the patient's age or weight. Preferably, the charging baseline (charging level) should be provided with a charging baseline for adults and charging baseline for children, and the user can take the patient's age or weight into consideration at the beginning of use so that either the charging baseline for adults and charging baseline for children can be configured.

The ECG analysis step proceeds simultaneously with the charging step after the initialization step (S110). The operation processing unit (120) analyzes the ECG signal that has been received from A/D conversion unit in order to obtain certain parameters such as the RR interval and QRS power spectrum, and these parameters are applied to determine whether the ECG is indicating ventricular fibrillation, arrhythmia (tachycardia, bradycardia, etc.), or normal heart activity (S130).

The operation processing method for determining whether an ECG is indicating ventricular fibrillation, arrhythmia (tachycardia, bradycardia, etc.), or normal heart activity by applying ECG parameters is well known, and thus a detailed description thereof will not be provided here.

In the ECG analysis step, noise generated from high-voltage charging by proceeding simultaneously with the charging step is removed from the ECG signal received from the A/D conversion unit (117), and the ECG signal from which the noise has been removed is analyzed to primarily determine the presence of ventricle defibrillation.

The charging step (S120) and the ECG analysis step (S130) are performed simultaneously. and these two steps may be collectively referred to as an ECG analysis and charging step (S115).

In the electric shock point determination step (S150), the operation processing unit (120) will judge that current time point is not the right time to deliver an electric shock (S150) if it is determined in the ECG analysis step that there was no ventricular fibrillation, and it will proceed with the discharging step (S200). If it is determined in the ECG analysis step that there was ventricular fibrillation, the current time point is determined as the right timing for an electric shock, the electric shock flag is set and the electric shock guidance is provided through the sound output unit (200) before proceeding with the shock cancellation determination step (S160).

In the shock cancellation determination step (S160), if it has been determined that it is the right timing for an electric shock in the electric shock timing determination step (that is, if it is determined that it is the electric shock time point), it will be determined whether ventricular fibrillation is indicated on the ECG from the electric shock timing determination step to the present moment (immediately before the shock cancellation determination step) (S160), and if it is still determined that it is a case of ventricular fibrillation, it will proceed to the electric shock step (S170), but if it is determined that it is not a case of ventricular fibrillation, the electric shock flag will be reset (i.e. cancellation of the electric shock) and the cancellation counter will be increased by one (S180) before proceeding with the three successive cancellation determination step (S190).

The shock cancellation determination step (S160) is intended to prevent a case in which an electric shock is delivered when unnecessary as the ECG signal has returned to normal while an electric shock guidance is being given after the electric shock timing determination step. An electric shock cancellation indicates that the patient's condition is close to the boundary at which electric shock is required.

The electric shock stage (S170) is a case in which electric shock is required as it is judged in the shock cancellation determination step (S160) that there is ventricular fibrillation. The operation processing unit sends an electric shock signal to the electric shock power unit (170) so that the electric shock power unit (170) applies an electric shock to the patient through the defibrillator electrode unit (175) the energy that has been charged, and when the electric shock is over, the electric shock flag is reset before moving on to the CPR step (S210).

In the three successive cancellation determination step (S190), if an electric shock has been canceled, as it was judged in the shock cancellation determination step (S160) that there was no ventricular fibrillation, it is determined whether the electric shock cancellation occurred three times in succession (i.e. the cancellation count is 3), and if it indeed occurred three times in succession, it will proceed to the discharge step (S200), but if it did not occur three times in succession (i.e. the cancellation count is not 3), it will proceed to the charging step (S120) and ECG analysis step (S130) - that is, the ECG analysis and charging step (S115) -- and the charging step (S120) and ECG analysis step (S130) will proceed simultaneously.

In the three successive cancellation determination step (S190), if the electric shock was cancelled fewer than three times in succession, there is a high possibility of applying an electric shock again. Thus, in this case, no discharge is performed, and proceeds to the ECG analysis and charging step (S115). By doing this, it is on standby to deliver an electric shock at any time, and it can shorten the time required for charging, which in turn raises the probability of resuscitating the patient.

In the discharging step (S200), if it is judged in the electric shock timing determination step that it is not the right time for an electric shock, or it is judged in the three successive cancellation determination step (S190) that electric shock cancellation has occurred three times in succession, the operation processing unit (120) will generate a signal to discharge and transmit it to the energy discharging unit (165) for the energy discharging unit (165) to discharge, and then the device will proceed to the cardiopulmonary resuscitation step (S210).

In the CPR step (S210), if an electric shock has been completed in the electric shock stage (S170), or the signal to discharge is transmitted to the energy discharging unit (165) in the discharge stage (S200), the operation processing unit will set the flag of the CPR time, wait until the CPR time duration is over, reset the flag of the CPR time when the CPR time duration is over, and proceed to the charging step (S120) and ECG analysis step (S130) - that is, the ECG analysis and charging step (S115) -- and the charging step (S120) and ECG analysis step (S130) will proceed simultaneously.

The present disclosure provides a method which is not part of the invention of reducing the time it takes from the first analysis to the application of an electric shock by simultaneously performing charging and analysis. The ECG signal filter unit (not shown) of the present invention, in particular, is configured to filter noise generated during high-voltage charging, and thus even when charging and ECG analysis are performed simultaneously, the noise generated during high-voltage charging does not affect the ECG analysis.

Generally, in the case of patients experiencing ventricular fibrillation, arrhythmia, cardiac arrest, etc., it takes a long time to perform CPR after setting up the defibrillator, and there is time consuming during an electric shock is applied after judging the need for delivering an electric shock and charging the device, which can have a significant impact on the probability of resuscitating the patient. In fact, proper emergency treatment may not be provided within the golden time. The present invention, in consideration of the above, relates to a method of performing ECG analysis and charging for an electric shock at the same time from the beginning, in order to resuscitate the patient as quickly as possible so that an electric shock can be applied immediately, as necessary.

Generally, the time required to turn on the defibrillator and charge it after the initial ECG analysis is about 20 seconds, but the present invention shortens it to around 10 seconds by proceeding with ECG analysis and charging at the same time. That is, in the past, it was possible to deliver an electric shock from about 20 seconds after the power was turned on, but the present invention is capable of delivering an electric shock from about 10 seconds after the power is turned on, which can increase the probability of resuscitating of an emergency patient.

In other words, the present invention is configured to perform ECG analysis and charging simultaneously even after CPR, thereby reducing the time it takes (to 10 seconds) until the next electrical shock. In addition, the voltage charged in the high voltage capacitor is not discharged immediately, even when the ECG waveform returns to normal during analysis or when an electrical shock is canceled due to a sudden change in bioimpedance, and is instead maintained so that energy is discharged via internal discharge in the event that the electric shock is canceled three consecutive times.

That is, when recharging, it is charged from the remaining voltage to the baseline, which helps reduce the time it takes to recharge. This can also reduce the charging time and battery consumption for charging.

As described above, the present invention has been described by limited embodiments and figures, but the present invention is not limited to the above embodiments, and various modifications and alterations from these descriptions can be made by a person with ordinary skill in the art to which the present invention pertains.

### [Industrial Utility]

The present invention is applied to a defibrillator for early defibrillation by enabling ECG analysis and high-voltage charging to be performed simultaneously from the moment the defibrillator is turned on, reducing the time it takes to deliver the initial electric shock and increasing the probability of resuscitating the patient.

## Claims

1. A defibrillator comprising:
an ECG detection unit configured to detect and amplify an ECG signal, remove noise and convert it into a digital signal; an operation processing unit configured to analyze the ECG signal received from the ECG detection unit to determine the presence of ventricular fibrillation and generate an electric shock signal if ventricular fibrillation is present; and an electric shock unit configured to apply high energy to a defibrillation electrode upon receiving an electric shock signal from the operation processing unit, thereby delivering an electric shock;
**characterised in that**
the operation processing unit is further configured to receive and analyze the ECG signal from the ECG detection unit as soon as the power switch of the key input unit is turned on and simultaneously transmit a signal to charge to the energy charging unit of the electric shock unit for the energy charging unit to charge the energy of the electric shock power unit to a preset baseline.

2. The defibrillator according to claim 1,
wherein the operation processing unit is further configured to analyze the ECG signal received from the ECG detection unit to first determine whether ventricular fibrillation is present, and if it is determined that there is ventricular fibrillation, to judge that it is time to deliver an electric shock and provide an electric shock delivery guidance through the speaker,
whereby the operation processing unit is further configured to determine whether there is ventricular fibrillation for a second time Z from the ECG signal during the time from the point at which an electric shock is judged necessary to the time point at which an electric shock delivery guidance is provided through the speaker,
whereby the operation processing unit is further configured such that if it is judged that there is ventricular fibrillation, an electric shock signal is generated and sent to the electric shock unit for the electric shock unit to deliver an electric shock.

3. The defibrillator according to claim 2,
wherein the operation processing unit is so configured, such that when it is judged that it is not ventricular fibrillation during the second assessment for presence of ventricular fibrillation, the operation processing unit cancels the prior judgment that an electric shock is necessary and determines whether such cancellation has occurred three times in succession, and if such cancellation has occurred three times in succession, the operation processing unit generates a signal to discharge and transmits it to the energy discharging unit for the energy discharging unit to discharge the power charged in the energy charging unit.

4. The defibrillator according to claim 3,
further configued, such that after an electric shock is performed by the electric shock unit, or after the operation processing unit transmits a signal to discharge to the energy discharge unit, the preset CPR time duration begins,
and the operation processing unit, after the CPR time duration ends, analyzes the ECG signal received from the ECG detection unit and, at the same time, transmits the signal to charge to the energy charging unit of the electric shock unit for the energy charging unit to charge the energy of the electric shock power unit to a preset baseline.

5. The defibrillator according to claim 4,
whereby the CPR time duration is 2 minutes.

6. The defibrillator according to claim 2,
further configured, such that
the operation processing unit first analyzes the ECG signal received from the ECG detection unit to first determine whether there is ventricular fibrillation and, if it is determined that it is not ventricular fibrillation, generates a signal to discharge to be transmitted to the energy discharge unit for the energy discharge unit to discharge the power charged in the energy charging section from the inside the equipment via internal discharge.

7. The defibrillator according to claim 4,
further configured, such that
the operation processing unit, when it is judged that it is not ventricular fibrillation during the second assessment for presence of ventricular fibrillation, cancels the prior judgment that an electric shock is necessary and determines whether such cancellation has occurred three times in succession, and if such cancellation has not occurred three times in succession;
the operation processing unit analyzes the ECG signal received from the ECG detection unit and, at the same time;
transmits the signal to charge to the energy charging unit of the electric shock unit for the energy charging unit to charge the energy of the electric shock power unit to a preset baseline.

8. The defibrillator according to claim 1,
wherein the ECG detection unit, for the purpose of simultaneously analyzing ECG signal and charging energy, further comprises an ECG signal filter unit configured to remove noise caused by high voltage charging from the ECG signal.

9. The defibrillator according to claim 1, or claim 4, or claim 7, further configured, such that the baseline may vary depending on whether the patient is a child or adult or the age or weight of the patient.

## Patentansprüche

1. Defibrillator, umfassend:
eine EKG-Erfassungseinheit, die konfiguriert ist, um ein EKG-Signal zu erfassen und zu verstärken, Rauschen zu entfernen und es in ein digitales Signal umzuwandeln; eine Betriebsverarbeitungseinheit, die konfiguriert ist, um das von der EKG-Erfassungseinheit empfangene EKG-Signal zu analysieren, um das Vorhandensein von Kammerflimmern zu bestimmen und ein elektrisches Schocksignal zu erzeugen, wenn Kammerflimmern vorliegt; und eine Elektroschockeinheit, die konfiguriert ist, um beim Empfang eines elektrischen Schocksignals von der Betriebsverarbeitungseinheit hohe Energie an eine Defibrillationselektrode anzulegen, wodurch ein elektrischer Schock abgegeben wird;
**dadurch gekennzeichnet, dass** die Betriebsverarbeitungseinheit ferner konfiguriert ist, um das EKG-Signal von der EKG-Erfassungseinheit zu empfangen und zu analysieren, sobald der Netzschalter der Tasteneingabeeinheit eingeschaltet wird, und gleichzeitig ein Signal zum Laden an die Energieladeeinheit der Elektroschockeinheit zu übertragen, damit die Energieladeeinheit die Energie der Elektroschock-Energieeinheit auf eine voreingestellte Basislinie auflädt.

2. Defibrillator nach Anspruch 1,
wobei die Betriebsverarbeitungseinheit ferner konfiguriert ist, um das von der EKG-Erfassungseinheit empfangene EKG-Signal zu analysieren, um zunächst zu bestimmen, ob Kammerflimmern vorliegt, und wenn bestimmt wird, dass Kammerflimmern vorliegt, zu beurteilen, dass es an der Zeit ist, einen elektrischen Schock abzugeben, und eine Anleitung zur Abgabe eines elektrischen Schocks über den Lautsprecher bereitzustellen, wobei die Betriebsverarbeitungseinheit ferner konfiguriert ist, um aus dem EKG-Signal während der Zeit zwischen dem Punkt, an dem ein Elektroschock als notwendig erachtet wird, und dem Zeitpunkt, an dem eine Anleitung zur Abgabe eines Elektroschocks über den Lautsprecher bereitgestellt wird, zu bestimmen, ob ein zweites Mal Kammerflimmern vorliegt,
wobei die Betriebsverarbeitungseinheit ferner so konfiguriert ist, dass, wenn beurteilt wird, dass ein Kammerflimmern vorliegt, ein Elektroschocksignal erzeugt und an die Elektroschockeinheit übertragen wird, damit die Elektroschockeinheit einen Elektroschock abgibt.

3. Defibrillator nach Anspruch 2,
wobei die Betriebsverarbeitungseinheit so konfiguriert ist, dass, wenn während der zweiten Einschätzung des Vorhandenseins von Kammerflimmern beurteilt wird, dass es sich nicht um Kammerflimmern handelt, die Betriebsverarbeitungseinheit die vorherige Beurteilung, dass ein elektrischer Schock notwendig ist, aufhebt und bestimmt, ob eine solche Aufhebung dreimal hintereinander aufgetreten ist, und wenn eine solche Aufhebung dreimal hintereinander aufgetreten ist, die Betriebsverarbeitungseinheit ein Signal zum Entladen erzeugt und es an die Energieentladungseinheit sendet, damit die Energieentladungseinheit die in der Energieladeeinheit geladene Energie entlädt.

4. Defibrillator nach Anspruch 3,
der ferner so konfiguriert ist, dass nach der Durchführung eines Elektroschocks durch die Elektroschockeinheit oder nachdem die Betriebsverarbeitungseinheit ein Signal zur Entladung an die Energieentladungseinheit überträgt, die voreingestellte CPR-Zeitdauer beginnt,
und die Betriebsverarbeitungseinheit nach dem Ende der CPR-Zeitdauer das von der EKG-Erfassungseinheit empfangene EKG-Signal analysiert und gleichzeitig das Signal zum Aufladen an die Energieladeeinheit der Elektroschockeinheit überträgt, damit die Energieladeeinheit die Energie der Elektroschock-Energieeinheit auf eine voreingestellte Basislinie auflädt.

5. Defibrillator nach Anspruch 4,
wobei die CPR-Zeitdauer 2 Minuten beträgt.

6. Defibrillator nach Anspruch 2,
der ferner so konfiguriert ist, dass die Betriebsverarbeitungseinheit zunächst das von der EKG-Erfassungseinheit empfangene EKG-Signal analysiert, um zunächst bestimmen, ob ein Kammerflimmern vorliegt, und, wenn bestimmt wird, dass kein Kammerflimmern vorliegt, ein Signal zum Entladen erzeugt, das an die Energieentladungseinheit übertragen werden soll, damit die Energieentladungseinheit die in dem Energieladeabschnitt geladene Energie aus dem Inneren des Geräts durch interne Entladung entlädt.

7. Defibrillator nach Anspruch 4,
der ferner so konfiguriert ist, dass die Betriebsverarbeitungseinheit, wenn während der zweiten Einschätzung des Vorhandenseins von Kammerflimmern bestimmt wird, dass es sich nicht um Kammerflimmern handelt, die vorherige Beurteilung, dass ein elektrischer Schock erforderlich ist, aufhebt und bestimmt, ob eine solche Aufhebung dreimal hintereinander erfolgt ist, und wenn eine solche Aufhebung dreimal hintereinander nicht erfolgt ist;
die Betriebsverarbeitungseinheit das von der EKG-Erfassungseinheit empfangene EKG-Signal analysiert und gleichzeitig;
das Signal zum Aufladen an die Energieladeeinheit der Elektroschockeinheit überträgt, damit die Energieladeeinheit die Energie der Elektroschock-Energieeinheit auf eine voreingestellte Basislinie auflädt.

8. Defibrillator nach Anspruch 1,
wobei die EKG-Erfassungseinheit zum Zweck der gleichzeitigen Analyse des EKG-Signals und der Ladeenergie ferner eine EKG-Signalfiltereinheit umfasst, die konfiguriert ist, um durch Hochspannungsladung verursachtes Rauschen aus dem EKG-Signal zu entfernen.

9. Defibrillator nach Anspruch 1, Anspruch 4 oder Anspruch 7,
der ferner so konfiguriert ist, dass die Basislinie je nachdem, ob es sich bei dem Patienten um ein Kind oder einen Erwachsenen handelt, oder je nach Alter oder Gewicht des Patienten variieren kann.

## Revendications

1. Défibrillateur, comprenant
une unité de détection ECG configurée pour détecter et amplifier un signal ECG, éliminer le bruit et le convertir en un signal numérique ; une unité de traitement d'opération configurée pour analyser le signal ECG reçu depuis l'unité de détection ECG afin de déterminer la présence d'une fibrillation ventriculaire et de générer un signal de choc électrique en cas de fibrillation ventriculaire ; et une unité de choc électrique configurée pour appliquer une énergie élevée à une électrode de défibrillation lors de la réception d'un signal de choc électrique depuis l'unité de traitement d'opération, administrant ainsi un choc électrique ;
**caractérisé en ce que** l'unité de traitement d'opération est configurée pour recevoir et analyser le signal ECG de l'unité de détection ECG, dès que l'interrupteur d'alimentation de l'unité d'entrée de clé est mis en marche ;
et transmettre simultanément un signal de charge à l'unité de charge d'énergie de l'unité de choc électrique pour que l'unité de charge d'énergie charge l'énergie de l'unité d'énergie de choc électrique jusqu'à une ligne de base prédéfinie.

2. Défibrillateur selon la revendication 1,
dans lequel l'unité de traitement d'opération est en outre configurée pour analyser le signal ECG reçu depuis l'unité de détection ECG afin de déterminer tout d'abord s'il y a une fibrillation ventriculaire et, s'il est déterminé qu'il y a une fibrillation ventriculaire, pour juger qu'il est temps d'administrer un choc électrique et fournir un guide d'administration de choc électrique par l'intermédiaire du haut-parleur, l'unité de traitement d'opération étant en outre configurée pour déterminer s'il y a une deuxième fois une fibrillation ventriculaire à partir du signal ECG pendant la période comprise entre le moment où un choc électrique est jugé nécessaire et le moment où un guidage d'administration de choc électrique est fourni par l'intermédiaire du haut-parleur,
l'unité de traitement d'opération étant en outre configurée de manière à ce que, si l'on juge qu'il y a une fibrillation ventriculaire, un signal de choc électrique soit généré et envoyé à l'unité de choc électrique pour que celle-ci administre un choc électrique.

3. Défibrillateur selon la revendication 2,
dans lequel l'unité de traitement d'opération est configurée de telle sorte que lorsqu'il est jugé qu'il ne s'agit pas d'une fibrillation ventriculaire au cours de la deuxième évaluation de la présence d'une fibrillation ventriculaire, l'unité de traitement d'opération annule le jugement préalable selon lequel un choc électrique est nécessaire et détermine si cette annulation s'est produite trois fois de suite, et si cette annulation s'est produite trois fois de suite, l'unité de traitement d'opération génère un signal de décharge et le transmet à l'unité de décharge d'énergie pour que l'unité de décharge d'énergie décharge l'énergie chargée dans l'unité de charge d'énergie.

4. Défibrillateur selon la revendication 3,
configuré en outre de manière à ce qu'après un choc électrique effectué par l'unité de choc électrique, ou après que l'unité de traitement d'opération a transmis un signal de décharge à l'unité de décharge d'énergie, la durée prédéfinie de la RCP commence à s'écouler,
et l'unité de traitement d'opération, après la fin de la durée de la RCP, analysant le signal ECG reçu depuis l'unité de détection ECG et, en même temps, transmettant le signal de charge à l'unité de charge d'énergie de l'unité de choc électrique pour que l'unité de charge d'énergie charge l'énergie de l'unité d'énergie de choc électrique à une ligne de base prédéfinie.

5. Défibrillateur selon la revendication 4,
la durée de la RCP étant de 2 minutes.

6. Défibrillateur selon la revendication 2,
configuré de manière à ce que l'unité de traitement d'opération analyse d'abord le signal ECG reçu depuis l'unité de détection ECG pour déterminer s'il y a une fibrillation ventriculaire et, s'il est déterminé qu'il ne s'agit pas d'une fibrillation ventriculaire, génère un signal de décharge à transmettre à l'unité de décharge d'énergie pour que l'unité de décharge d'énergie décharge l'énergie chargée dans la section de charge d'énergie à l'intérieur de l'équipement via une décharge interne.

7. Défibrillateur selon la revendication 4,
configurée en outre de manière à ce que l'unité de traitement d'opération, lorsqu'elle juge qu'il ne s'agit pas d'une fibrillation ventriculaire au cours de la deuxième évaluation de la présence d'une fibrillation ventriculaire, annule le jugement préalable selon lequel un choc électrique est nécessaire et détermine si cette annulation s'est produite trois fois de suite, et si cette annulation ne s'est pas produite trois fois de suite ;
l'unité de traitement d'opération analysant le signal ECG reçu depuis l'unité de détection ECG et, en même temps ;
transmettant le signal de charge à l'unité de charge d'énergie de l'unité de choc électrique pour que l'unité de charge d'énergie charge l'énergie de l'unité d'énergie de choc électrique jusqu'à une ligne de base prédéfinie.

8. Défibrillateur selon la revendication 1,
dans lequel l'unité de détection ECG, dans le but d'analyser simultanément le signal ECG et l'énergie de charge, comprend en outre une unité de filtrage de signal ECG configurée pour éliminer du signal ECG le bruit causé par la charge à haute tension.

9. Défibrillateur selon la revendication 1, ou la revendication 4, ou la revendication 7,
configuré de manière à ce que la ligne de base puisse varier selon que le patient est un enfant ou un adulte, ou selon l'âge ou le poids du patient.
